# DEMANDE DE BREVET EUROPEEN

(11) **EP 4 299 566 A1**
(43) Date de publication de la demande: **03.01.2024**
(21) Numéro de dépôt: 22182679.5
(22) Date de dépôt: 01.07.2022
(51) Int. Cl.: C07D 307/46, C07D 307/48, B01J 29/70

(54) **PROCEDE DE PRODUCTION DE 5-HYDROXYMETHYLFURFURAL**

(71) Demandeur: IFP Energies nouvelles, 92500 Rueil-Malmaison (FR)
(72) Inventeur: LARMIER, Kim, 92852 Rueil-Malmaison Cedex (FR); PIRNGRUBER, Gerhard, 92852 Rueil-Malmaison Cedex (FR); HAN, Yuna, 92852 Rueil-Malmaison Cedex (FR)
(74) Mandataire: IFP Energies nouvelles

(57) **Abrégé**

La présente invention concerne un procédé de déshydratation d'une charge comprenant un hexose, ledit procédé comprenant les étapes de :
- Préparation d'un catalyseur par mise en contact d'une zéolithe comprenant une série de canaux dont l'ouverture est supérieure ou égale à 10 atomes d'oxygène (10MR) et présentant un rapport Si/Al d'au moins 10, avec une solution comprenant un composé organique de formule : 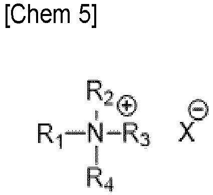 R₁, R₂, R₃ et R₄ étant choisis indépendamment parmi les groupements alkyles linéaires, branchés ou cycliques, de 1 à 10 atomes de carbone, et X- étant l'ion hydroxyde OH- ;
- Mise en contact dans au moins un solvant dudit catalyseur de déshydratation avec ladite charge comprenant un hexose, ladite mise en contact étant opérée à une température comprise entre 30 et 160°C et à une pression comprise entre 0,0001 et 8,0 MPa.

## Description

### DOMAINE TECHNIQUE

L'invention concerne un procédé de production de 5-hydroxyméthylfurfural (5-HMF) à partir d'une charge contenant des sucres, en particulier au moins un hexose et de préférence du fructose, en présence d'au moins un solvant, de préférence polaire aprotique, et d'au moins un catalyseur de déshydratation comprenant une zéolithe modifiée par un traitement basique en présence de composés organiques appartenant à la famille des hydroxydes de tétraalkylammonium.

### TECHNIQUE ANTERIEURE

Le 5-hydroxyméthylfurfural (5-HMF) est un composé dérivé de la biomasse qui peut être valorisé de nombreuses façons, comme dans la chimie de spécialité, les polymères, ou l'agrochimie.

La production de 5-HMF par déshydratation d'hexoses est bien connue de l'homme du métier et fait l'objet d'intenses recherches. Il est connu que l'utilisation d'un catalyseur acide de Brønsted accélère la réaction. Les catalyseurs de la classe des zéolithes sont notamment capables de catalyser efficacement la réaction.

Cependant, la diffusion des hexoses dans la porosité des zéolithes peut s'avérer limitante pour la cinétique de la réaction tel que le décrit le document Dapsens et al. ((2014) Hierarchical Sn-MFI zeolites prepared by facile top-down methods for sugar isomerisation. In : Catalysis Science & Technology, vol. 4, n° 8, p. 2302). Par ailleurs, comme l'enseigne le document Ordomsky et al. ((2012) The effect of solvent addition on fructose dehydration to 5-hydroxymethylfurfural in biphasic system over zeolites. In : Journal of Catalysis, vol. 287, p. 68-75), la mise en contact prolongée des sucres avec la surface externe des zéolithes entraîne la formation de sous-produits polymériques indésirables, appelés de manière générique humines, qui amènent à la fois à des pertes de rendement et à l'inactivation du catalyseur.

Il est donc avantageux de réduire les limitations diffusionnelles des hexoses dans les zéolithes.

Le document Rac et al. ((2014) Hierarchical ZSM-5, Beta and USY zeolites. Acidity assessment by gas and aqueous phase calorimetry and catalytic activity in fructose dehydration reaction. In : Microporous and Mesoporous Materials, vol. 194, p. 126-134), divulgue l'utilisation de zéolithes modifiées par un traitement basique en présence de soude (NaOH) et de bromure de tétrapropylammonium dans un procédé de déshydratation du fructose en 5-HMF.

L'inconvénient des procédés de l'art antérieur est l'utilisation de NaOH conjointement aux composés tétraalkylammonium lors des traitements de modification des zéolithes. En effet, l'utilisation de NaOH (ou d'autres hydroxydes d'alcalins ou alcalino-terreux) neutralise l'acidité de la zéolithe et nécessite des étapes supplémentaires de retroéchange de Na+ (ou d'autres sels alcalins ou alcalino-terreux) après le traitement basique, ces étapes rendant le procédé onéreux.

Ainsi, la présente invention vise à améliorer les performances des catalyseurs zéolithiques pour la production de 5-HMF à partir de sucres, tout en limitant les coûts. La demanderesse a en effet découvert qu'il est possible d'utiliser, comme catalyseurs de la conversion des sucres en 5-HMF, des zéolithes modifiées par un traitement en milieu basique en présence d'au moins un composé organique agissant comme agent directeur de structure appartenant à la catégorie des hydroxydes de tétraalkylammonium sans utiliser de composé basique additionnel, tel que la soude, afin d'améliorer les performances catalytiques de production de 5-HMF à partir d'hexoses. Avantageusement, la sélectivité est améliorée et la formation de sous-produit oligomérique est limitée, et le procédé est plus économique.

### RESUME DE L'INVENTION

La présente invention concerne un procédé de déshydratation d'une charge comprenant un hexose, ledit procédé comprenant les étapes de :
- Préparation d'un catalyseur par mise en contact d'une zéolithe comprenant une série de canaux dont l'ouverture est supérieure ou égale à 10 atomes d'oxygène (10MR) et présentant un rapport Si/AI d'au moins 10, avec une solution comprenant un composé organique de formule : 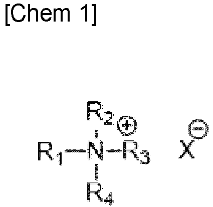 R₁, R₂, R₃ et R₄ étant choisis indépendamment parmi les groupements alkyles linéaires, branchés ou cycliques, de 1 à 10 atomes de carbone, et X- étant l'ion hydroxyde OH- ;
- Mise en contact dans au moins un solvant dudit catalyseur de déshydratation avec ladite charge comprenant un hexose, ladite mise en contact étant opérée à une température comprise entre 30 et 160°C et à une pression comprise entre 0,0001 et 8,0 MPa.

### DESCRIPTION DETAILLEE DE L'INVENTION

Selon la présente invention, l'expression « compris entre ... et ... » et « entre .... et ... » sont équivalentes et signifient que les valeurs limites de l'intervalle sont incluses dans la gamme de valeurs décrite. Si tel n'est pas le cas et que les valeurs limites ne sont pas incluses dans la gamme décrite, une telle précision sera apportée par la présente invention.

Dans le sens de la présente invention, les différentes plages de paramètres pour une étape donnée telles que les plages de pression et les plages de température peuvent être utilisées seules ou en combinaison. Par exemple, dans le sens de la présente invention, une plage de valeurs préférées de pression peut être combinée avec une plage de valeurs de température plus préférée.

Dans la suite, des modes de réalisation particuliers de l'invention peuvent être décrits. Ils pourront être mis en œuvre séparément ou combinés entre eux, sans limitation de combinaisons lorsque c'est techniquement réalisable.

### Charge

La charge mise en œuvre dans le procédé selon l'invention comprend un hexose.

On entend par la charge qui comprend un hexose que l'hexose peut être sous forme monomérique (monosaccharide) ou alors être une unité appartenant à un disaccharide, oligosaccharide ou polysaccharide. Un saccharide est un composé appelé encore sucre.

De manière préférée l'hexose est le fructose ou une unité fructosidique.

Dans un mode de réalisation, la charge comprend du fructose libre, seul ou en mélange avec toute espèce saccharidique, soit comprend toute charge oligosaccharide ou polysaccharidique contenant une ou plusieurs unités fructosidique(s) pouvant libérer du fructose par une ou plusieurs étapes d'hydrolyse, éventuellement en mélange avec d'autres espèces saccharidiques. Préférentiellement, la charge traitée dans le procédé est du fructose cristallin, un sirop contenant du fructose et du glucose, ou le saccharose cristallin ou un sirop de saccharose.

Avantageusement, la charge comprend du fructose sous forme monomérique, oligomérique ou polymérique.

Par charge contenant du fructose libre pris en mélange avec toute espèce saccharidique, on désigne par exemple des sirops de type High-Fructose-Corn-Syrup contenant du fructose et du glucose en différentes proportions (glucose/fructose dans des rapports massiques ou molaires 58/42, 45/55, 10/90 par exemple).

On entend par sirop, une solution de sucres, en particulier de saccharides, dans l'eau ayant une concentration d'au moins 30 % en poids, de préférence au moins 50 % en poids, de préférence au moins 70 % en poids.

La charge peut comprendre un saccharide comprenant une ou plusieurs unités fructosidiques et une ou plusieurs unités non-fructosidiques, du fructose pouvant être libéré par une ou plusieurs étapes d'hydrolyse, par exemple les oligosaccharides et les polysaccharides dans lesquels au moins une unité monosaccharidique est le fructose, par exemple des charges telles que le saccharose, le kestose, les fructanes, les oligofructanes, l'inuline.

Avantageusement, les charges saccharidiques sont aptes à libérer du fructose monomérique par hydrolyse osidique, ledit fructose produit pouvant être transformé en 5-HMF.

De préférence, l'oligosaccharide a pour formule brute : (C₆ₘH₁₀ₘ₊₂O₅ₘ₊₁) (C₅ₙH₈ₙ₊₂O₄ₙ₊₁) où m et n sont des entiers dont la somme est comprise entre 2 et 6. Les unités monosaccharidiques composant ledit oligosaccharide sont identiques ou non, et au moins une unité de formule (C₆ₘH₁₀ₘ₊₂O₅ₘ₊₁) est le fructose. Par extension, de préférence le polysaccharide a pour formule brute (C₆ₘH₁₀ₘ₊₂O₅ₘ₊₁) (C₅ₙH₈ₙ₊₂O₄ₙ₊₁) où m et n sont des entiers dont la somme est supérieure ou égale à 7.

### Préparation du catalyseur de déshydratation

Le catalyseur de déshydratation est préparé à partir d'une zéolithe qui comprend une série de canaux dont l'ouverture est supérieure ou égale à 10 atomes d'oxygène (10MR) et présente un rapport molaire Si/Al d'au moins 10.

Avantageusement, cette zéolithe de départ est appelée zéolithe mère.

Dans un mode de réalisation, l'ouverture de la série de canaux de la zéolithe est égale à 12 atomes d'oxygène (12MR).

De préférence il s'agit d'une zéolithe appartenant au type structurale FAU ou EMT. De manière très préférée, il s'agit d'une faujasite désaluminée, appelée USY (Ultra Stable Y), avantageusement avec un paramètre de maille inférieur à 24,5 Â.

Dans un mode de réalisation préféré, le rapport molaire Si/Al de la zéolithe mère est compris entre 10 de 50, de préférence entre 12 et 35.

Si le rapport molaire Si/Al de la zéolithe mère est inférieur à 10, on peut appliquer un traitement de désalumination, de préférence préalablement à toute étape de mise en contact avec un composé organique de type hydroxyde de tétraalkylammonium, afin d'obtenir une zéolithe avec un rapport molaire Si/Al dans la gamme souhaitée. Ce traitement de désalumination consiste à une mise en contact de la zéolithe avec une solution acide ou une solution comprenant un agent complexant, capable de former un complexe stable avec Al³⁺ en solution. Ces méthodes sont bien connues de l'homme de l'art. Quelques exemples d'acides bien adaptés sont : HNO₃, HCl, H₂SO₄, CH₃COOH, HOOC-COOH. Comme agent complexant on peut citer l'EDTA (l'acide éthylène diamine tétra-acétique), sous sa forme acide ou de sel de Na, le NTA (l'acide nitrilotriacétique), etc. La concentration de l'acide ou de l'agent complexant dans la solution acide ou comprenant un agent complexant est de préférence comprise entre 0,1 et 6 mol/l. Le volume de solution utilisé est de préférence compris entre 3 et 50 ml par gramme de zéolithe (masse sèche de la zéolithe). La température du traitement de désalumination est de préférence comprise entre 20 et 100°C. La durée du traitement de désalumination est préférentiellement comprise entre 15 min et 10 h. Après le traitement de désalumination, la zéolithe est avantageusement séparée par filtration ou centrifugation, séchée, de préférence à une température entre 80 et 150°C, et finalement calcinée à une température entre 450°C et 600°C.

On entend par masse sèche d'une zéolithe : la masse de ladite zéolithe après traitement à 1000°C.

Pour améliorer la performance catalytique lors de la conversion des sucres, de manière préférée du fructose, en 5-HMF, la zéolithe (éventuellement après ajustement du rapport Si/AI par désalumination) est mise en contact avec une solution comprenant un composé organique de formule :

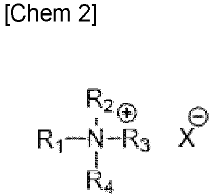

R₁, R₂, R₃ et R₄ étant choisis indépendamment parmi les groupements alkyles linéaires, branchés ou cycliques de 1 à 10 atomes de carbone, et X- étant l'ion hydroxyde OH⁻.

Ledit composé organique est plus précisément appelé un hydroxyde de tétraalkylammonium.

Les groupements R₁, R₂, R₃ et R₄ peuvent être identiques ou différents.

Dans un mode de réalisation particulier, ledit composé organique comprend au moins un groupement R₁, R₂, R₃, R₄, choisi parmi les groupements méthyl, éthyl, propyl, et butyl. Dans un mode de réalisation préféré, le composé organique est choisi parmi l'hydroxyde de tétraméthylammonium, l'hydroxyde de tétraéthylammonium, l'hydroxyde de tétrapropylammonium et l'hydroxyde de tétrabutylammonium.

Dans un mode de réalisation préféré, ledit composé organique comprend au moins un groupement R₁, R₂, R₃, R₄, choisi parmi les groupements propyl et butyl.

Dans un mode de réalisation très préféré, le composé organique est choisi parmi l'hydroxyde de tétrapropylammonium et l'hydroxyde de tétrabutylammonium.

Ce traitement de la zéolithe, par contact avec un tétraalkylammonium, permet de modifier l'acidité de la zéolithe et de générer des mésopores qui sont favorables à la diffusion des réactifs et des produits de la réaction de déshydratation des sucres.

Avantageusement, le composé organique est en solution, de préférence en solution aqueuse, lors de la mise en contact avec la zéolithe. De préférence, ladite solution ne comprend pas d'hydroxydes d'alcalins ou alcalino-terreux, comme NaOH. L'utilisation de NaOH ou d'autres hydroxydes d'alcalins ou alcalino-terreux, neutralise l'acidité de la zéolithe et nécessite des étapes supplémentaires de retroéchange de Na+ (ou sels alcalins ou alcalino-terreux) après le traitement basique, ces étapes supplémentaires rendant le procédé onéreux.

Dans un mode de réalisation particulier, ladite solution peut comprendre un second composé organique de formule : 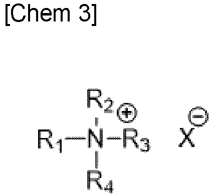 dans lequel R₁, R₂, R₃ et R₄ sont choisis indépendamment parmi les groupements alkyles linéaires, branchés ou cycliques, de 1 à 10 atomes de carbone et X⁻ est choisi parmi les ions halogénures Cl⁻, Br⁻, I⁻.

Il est possible d'employer un mélange de plusieurs cations N(R₁R₂R₃R₄) ⁺, ainsi qu'un mélange de plusieurs anions X⁻.

Il est important dans le procédé selon l'invention de distinguer l'anion OH⁻, qui apporte une basicité et permet la formation des mésopores au sein de la zéolithe mère, des autres anions tels que les halogénures.

Optionnellement, ladite solution peut comprendre une base organique, par exemple une amine tel que du diméthylamine ou triméthylamine, comme second composé organique.

La mise en contact de la zéolithe avec le composé organique et optionnellement le second composé organique est de préférence réalisée dans un contenant qui permet une agitation de la solution afin d'assurer un bon échange de matière.

Le volume de la solution est défini par rapport à la masse sèche de zéolithe utilisée (c'est-à-dire de zéolithe mère). On choisit en général un volume entre 3 et 100 ml par gramme de zéolithe, de préférence entre 5 et 50 ml par gramme de zéolithe.

La solution comprend généralement un solvant étant de l'eau, éventuellement mélangé avec un autre solvant protique et polaire.

La concentration de base de la solution, de la solution, correspond à la somme des quantités molaires de N(R₁R₂R₃R₄)OH plus celle du second composé organique optionnellement ajouté, par unité de volume de solution. Cette concentration de base est avantageusement comprise entre 0,1 et 2 mol/l, de préférence entre 0,1 et 0,3 mol/l.

Le rapport entre la concentration de N(R₁R₂R₃R₄)+ et la concentration totale de la base est compris entre 1 et 0,1.

De préférence, le pH final de la solution, c'est-à-dire, à la fin du traitement est compris entre 10 et 14.

Avantageusement, le traitement basique est réalisé à une température comprise entre 20 et 90°C, de préférence entre 50 et 70°C.

De préférence, la durée du traitement est comprise entre 15 min et 10h, de préférence entre 1 et 4 h.

A la fin du traitement, la zéolithe (appelée encore zéolithe traitée) est avantageusement séparée de la solution par filtration ou par centrifugation.

De préférence, la zéolithe issue du traitement (ou du dernier échange ionique) est séparée puis séchée à une température comprise entre 80 et 150°C (éventuellement en appliquant du vide) et finalement calcinée à une température comprise entre 500 et 650°C, dans un flux d'azote ou d'air. Pour des raisons de sécurité (pour éviter des réactions d'oxydation violentes du composé organique) il est possible de monter en température dans un flux d'azote, avant de basculer sur un flux d'air. La durée du palier de température finale est comprise entre 1 et 12 h.

Le catalyseur de déshydratation du procédé selon l'invention comprend la zéolithe traitée, obtenue à l'issue du traitement basique et de préférence séparée, séchée et calcinée. Ladite zéolithe obtenue à l'issue du traitement basique, et de préférence séparée, séchée et calcinée, peut éventuellement être mélangée avec un liant, pour constituer ledit catalyseur de déshydratation.

### Mise en œuvre de l'étape de déshydratation

Ledit procédé comprend une étape de déshydratation. Ladite étape de déshydratation des sucres est mise en œuvre par mise en contact de la charge qui comprend un hexose avec le catalyseur de déshydratation dans au moins un solvant, de préférence organique.

Le procédé selon l'invention est avantageusement un procédé de production de 5-hydroxyméthylfurfural à partir d'une charge comprenant un hexose.

L'étape de déshydratation du procédé selon l'invention, mise en œuvre par mise en contact de la charge qui comprend un hexose avec le catalyseur de déshydratation dans au moins un solvant, peut être opérée en continu ou en discontinu.

Ladite étape de mise en contact de la charge qui comprend un hexose avec le catalyseur de déshydratation dans au moins un solvant est opérée à une température comprise entre 30 et 160°C, de préférence entre 40 et 140°C, de préférence entre 50 et 120 °C, de manière préférée 80 et 120°C, de manière très préférée entre 90 et 120°C et à une pression comprise entre 0,0001 et 8,0 MPa, de préférence entre 0,001 et 5,0 MPa, et de manière préférée entre 0,01 et 3,0 MPa.

Le milieu réactionnel comprend la charge comprenant un hexose, au moins un solvant de préférence organique et le catalyseur de déshydratation.

De manière préférée le solvant est un solvant polaire aprotique. Le solvant polaire aprotique est avantageusement choisi parmi la butan-2-one, l'acétone, l'anhydride acétique, la *N,N,N',N'*-tétraméthylurée, le benzonitrile, l'acétonitrile, la méthyléthylcétone, le propionitrile, l'hexaméthylphosphoramide, le nitrobenzène, le nitrométhane, le *N,N*-diméthylformamide, le *N,N*-diméthylacétamide, le sulfolane, la *N*-méthylpyrrolidone, le diméthylsulfoxyde, le propylène carbonate et la g-valérolactone. De préférence, le solvant polaire aprotique est choisi parmi l'acétone, l'hexaméthylphosphoramide, *N,N*-diméthylformamide, le sulfolane, la *N*-méthylpyrrolidone, le diméthylsulfoxyde, le propylène carbonate et la g-valérolactone.

De manière très préférée, le solvant polaire aprotique est le diméthylsulfoxide (DMSO).

L'avantage d'utiliser un solvant polaire aprotique dans le procédé est que le 5-HMF produit ne se réhydrate pas, la réhydratation du 5-HMF dans un solvant tel que l'eau par exemple induit une perte de sélectivité du procédé vers le 5-HMF. L'utilisation d'un solvant polaire aprotique permet donc d'obtenir une sélectivité du procédé vers le 5-HMF optimale.

On entend par solvant aprotique, une molécule jouant le rôle de solvant et dont tous les atomes d'hydrogène sont portés par des atomes de carbone.

On entend par solvant polaire, une molécule jouant le rôle de solvant dont le moment dipolaire µ exprimé en Debye a une valeur numérique supérieure ou égale à 2,00 à 25°C, d'après les bases de données connues de l'Homme du métier.

On entend donc par solvant polaire aprotique, une molécule jouant le rôle de solvant dont tous les atomes d'hydrogène sont portés par des atomes de carbone et dont le moment dipolaire µ exprimé en Debye a une valeur numérique supérieure ou égale à 2,00 à 25°C, d'après les bases de données connues de l'Homme du métier.

La charge est introduite dans le milieu réactionnel dans un rapport massique solvant/charge compris entre 0,1 et 200,0, de préférence entre 0,3 et 100,0 et encore préférentiellement entre 1,0 et 50,0.

Ladite étape de déshydratation est avantageusement alimentée par la charge comprenant un hexose, sous forme solide ou liquide. L'alimentation de la charge peut être effectuée selon plusieurs modes d'introduction de ladite charge.

Dans un premier mode de réalisation, la charge est introduite sous forme solide, éventuellement à l'aide d'un dispositif adapté permettant de contrôler le débit de charge. De manière non limitative, ce dispositif peut être une vis sans fin ou un système pneumatique de transport de particules solides. De manière non limitative, ce mode de réalisation est préféré pour une charge de type oligosaccharide ou polysaccharide.

L'introduction d'une charge sous forme solide correspondant à du saccharose, à du kestose ou à de l'inuline à partir desquels le fructose est libéré par hydrolyse est une possibilité. Ladite introduction peut être réalisée en une ou plusieurs fois, de manière séquentielle ou bien en continu.

Dans un second mode de réalisation, la charge est introduite sous forme liquide, en solution dans un solvant, appelé solvant additionnel, à l'aide d'une pompe permettant de contrôler le débit d'introduction de la solution contenant la charge. Le solvant additionnel permet de dissoudre les sucres (ou saccharides) de la charge.

Le solvant additionnel peut être choisi parmi les solvants polaires aprotiques ou protiques. De préférence ledit solvant additionnel est choisi parmi la butan-2-one, l'acétone, l'anhydride acétique, la *N,N,N',N'*-tétraméthylurée, le benzonitrile, l'acétonitrile, la méthyléthylcétone, le propionitrile, l'hexaméthylphosphoramide, le nitrobenzène, le nitrométhane, le *N,N-*diméthylformamide, le *N,N*-diméthylacétamide, le sulfolane, la *N*-méthylpyrrolidone, le diméthylsulfoxyde, le propylène carbonate, la g-valérolactone, l'eau, le méthanol, l'éthanol, l'acide formique et l'acide acétique.

De préférence, le solvant additionnel choisi parmi les solvants polaires aprotiques ou protiques est l'acétone, l'hexaméthylphosphoramide, *N,N*-diméthylformamide, le sulfolane, la *N*-méthylpyrrolidone, le diméthylsulfoxyde, le propylène carbonate, la g-valérolactone l'eau, le méthanol et l'éthanol, de manière préférée parmi le *N,N*-diméthylformamide, le sulfolane, la *N*-méthylpyrrolidone, le diméthylsulfoxyde, l'eau et le méthanol, et de manière très préférée le solvant additionnel est choisi parmi l'eau et le diméthylsulfoxyde.

Dans ce mode de réalisation dans lequel un solvant additionnel est présent, la concentration finale en 5-HMF ne peut excéder la limite de solubilité du sucre dans le solvant additionnel, affectée du rapport de masse molaire entre le fructose et le 5-HMF, et affecté de la dilution de la charge par la masse de solvant polaire aprotique. Le choix du solvant additionnel est donc primordial pour obtenir une concentration finale de 5-HMF élevée.

La teneur du au moins un solvant additionnel est comprise entre 0 et 50% en poids, de préférence entre 0 et 35% en poids, de manière préférée entre 0 et 25% en poids, et de manière très préférée entre 0 et 20% en poids par rapport au poids total de solvant dans le milieu réactionnel.

Ce mode de réalisation est particulièrement bien adapté à une charge de type monosaccharide, voire oligosaccharide, qui peuvent être dissoutes dans le solvant additionnel à de fortes concentrations.

De préférence, l'introduction progressive d'une charge correspondant à un sirop de fructose ou un sirop de fructose et de glucose (de type High-Fructose-Corn-Syrup selon l'appellation anglophone) par l'intermédiaire d'une pompe est mise en œuvre. Ladite introduction peut être réalisée en une ou plusieurs fois, de manière séquentielle ou bien en continu, de manière à ce que la concentration instantanée en fructose est maintenue inférieure ou égale à 5,0 % en poids.

On entend par concentration en 5-HMF ou fructose, le rapport entre la masse de 5-HMF ou fructose, respectivement, et la masse de milieu réactionnel.

Dans une mise en œuvre en continu du procédé selon l'invention, la vitesse massique horaire (débit de charge massique/masse de catalyseurs) est comprise entre 0,01 h⁻¹ et 5,0 h⁻¹ et de préférence entre 0,02 h⁻¹ et 2,0 h⁻¹.

Quel que soit le mode de réalisation mis en œuvre pour l'étape de déshydratation du procédé, l'eau générée et donc contenue dans le mélange réactionnel est de préférence éliminée, par toutes méthodes connues de l'homme du métier, de préférence de façon continue, afin de maintenir une teneur en eau inférieure à 30,0 % en poids par rapport à la masse totale de solvant, de préférence inférieure à 20,0 % en poids, de manière préférée inférieure à 15,0 % en poids, et de manière très préférée inférieure à 10,0 % en poids.

Avantageusement la mise en œuvre du procédé de production de 5-HMF, notamment par le contrôle de la concentration instantanée, et conjointement à l'utilisation d'une zéolithe modifiée selon l'étape de préparation du catalyseur selon l'invention permet d'obtenir une bonne conversion du fructose engagé, ainsi qu'une excellente sélectivité en faveur du 5-HMF et d'améliorer la productivité en 5-HMF.

Ainsi, les sélectivité, rendement et productivité obtenus par la mise en œuvre du procédé selon l'invention permettent par exemple d'atteindre des concentrations massiques finales en 5-HMF supérieures à 3,5 % en poids. Le procédé selon l'invention permet avantageusement d'atteindre des concentrations massiques finales en 5-HMF supérieures à 5,0 % en poids, de préférence supérieures à 10 % en poids, et de manière très préférée supérieures à 15 % en poids par rapport au poids du milieu réactionnel.

### Les produits obtenus et leur mode d'analyse

Le produit obtenu sélectivement par le procédé de transformation selon l'invention est le 5-hydroxyméthylfurfural (5-HMF). A l'issue de la réaction mise en œuvre dans le procédé selon l'invention, le milieu réactionnel est analysé par chromatographie liquide haute performance (HPLC) pour quantifier les composés présents dans le milieu réactionnel, comme le fructose, le 5-HMF mais aussi des produits non désirés comme l'acide lévulinique, l'acide formique et tout coproduit contenant des sucres. La conversion de la charge et la teneur en fructose et en 5-HMF sont ensuite calculés.

Les humines sont quantifiées par différence de bilan carbone avec le carbone introduit initialement.

### EXEMPLES

Dans les exemples ci-dessous, le fructose utilisé comme charge est un fructose commercial et utilisé sans purification supplémentaire.

Le diméthylsulfoxyde, noté DMSO dans les exemples, utilisé comme solvant polaire aprotique, est commercial et utilisé sans purification supplémentaire.

Les concentrations massiques des constituants des mélanges réactionnels sont déterminées par chromatographie liquide haute performance (HPLC). Des prélèvements d'aliquots du mélange réactionnel, i.e. des échantillons, sont réalisés à intervalles réguliers pour en évaluer la composition par HPLC. Les manques à 100% pour les rendements en HMF sont liés soit à une conversion partielle du fructose, soit à la formation de produits non désirés (humines, espèces polymériques...).

On définit le taux de conversion du fructose (Conv_{FRU}) comme le rapport de la concentration molaire de fructose converti et de la concentration molaire en unités fructosidiques présentes dans la charge initiale, exprimé en %.

On définit le rendement en 5-HMF (Rdt_{HMF}) comme le rapport de la concentration molaire en 5-HMF mesurée dans les échantillons et de la concentration molaire en unités fructosidiques seulement présentes dans la charge initiale, exprimé en %.

### Exemple 1 : Préparation des catalyseurs de déshydratation

Les différents échantillons sont préparés selon le même mode opératoire, seule la zéolithe mère et la nature de l'hydroxyde de tétralkylammonium changent.

La zéolithe mère CBV720 utilisée possède un rapport molaire Si/AI de 15,7 et un paramètre de maille de 24,293 Â.

La zéolithe mère CBV760 utilisée possède un rapport molaire Si/AI de 27,2 et un paramètre de maille de 24,259 Â.

300 ml d'une solution aqueuse d'hydroxyde de tétralkylammonium à 0,2 M est préparée avec de l'eau distillée. Lorsque la solution atteint les 65 °C, 10 g de zéolithe mère sont introduits et agités à 400 tr/min pendant 2 h à 65 °C. La solution est trempée dans l'eau glacée, filtrée et lavée 3 à 4 fois avec 300 ml d'eau distillée et centrifugée à 8 000-10000 tr/min pendant 3 min. Le solide récupéré est séché dans une étuve à 120°C pendant une nuit. Le solide séché est broyé et calciné à 550 °C pendant 4 h (1 °C min⁻¹) après 2 heures de repos à 200 °C (1,5 °C min⁻¹).

Un solide comparatif est préparé selon l'art antérieur (BC), avec comme zéolithe mère une CBV760 et un traitement avec du NaOH et du bromure de tétrapropylammonium.

La zéolithe mère et le composé organique utilisé pour chaque préparation d'échantillon est récapitulé dans le tableau suivant :

**[Table 1]**

| Solide | Zéolithe mère | Composé organique (tétralkylammonium) |
|---|---|---|
| A1 | CBV720 | Hydroxyde de tétramethylammonium |
| A2 | CBV720 | Hydroxyde de tétraethylammonium |
| A3 | CBV720 | Hydroxyde de tétrapropylammonium |
| A4 | CBV720 | Hydroxyde de tétrabutylammonium |
| B1 | CBV760 | Hydroxyde de tétramethylammonium |
| B2 | CBV760 | Hydroxyde de tétraethylammonium |
| B3 | CBV760 | Hydroxyde de tétrapropylammonium |
| BC | CBV760 | Bromure de tétrapropylammonium |
| B4 | CBV760 | Hydroxyde de tétrabutylammonium |

Les caractéristiques des différentes zéolithes mères et échantillons calculées (Surface spécifique, Volume microporeux, Volume mésoporeux, cristallinité et acidité de Bronsted) sont présentées dans le tableau suivant :

**[Table 2]**

| solide | S_{BET} (m² g⁻¹) | Vmicro (cm³ g⁻¹) | Vₘₑₛₒ ^{b} (cm³ g⁻¹) | Vₜₒₜₐₗ (cm³ g⁻¹) | Cristallinité relative | Acidité Bronsted (µmol/g) | Acidité Lewis |
|---|---|---|---|---|---|---|---|
| A CBV720 | 885 | 0.251 | 0.277 | 0.528 | 100% | 196 | 141 |
| A1 | 800 | 0,164 | 0,667 | 0,831 | 67% | 194 | 206 |
| A2 | 834 | 0,228 | 0,453 | 0,681 | 94% | 198 | 104 |
| A3 | 903 | 0.261 | 0.364 | 0.625 | 107% | 230 | 83 |
| A4 | 904 | 0.268 | 0.311 | 0.579 | 107% | 196 | 59 |
| B CBV760 | 859 | 0.251 | 0.322 | 0.573 | 100% | 125 | 41 |
| B1 | 895 | 0.224 | 0.713 | 0.936 | 0% | 78 | 119 |
| B2 | 882 | 0.249 | 0.4 | 0.650 | 58% | 129 | 108 |
| B3 | 946 | 0,235 | 0,781 | 1,016 | 92% | 174 | 52 |
| BC | 1219 | 0,289 | 0,991 | 1,280 | 68% | 76 | 38 |
| B4 | 882 | 0,249 | 0,400 | 0,650 | 100% | 153 | 56 |

| | | | | | | | |
|---|---|---|---|---|---|---|---|
| N.D = Non Défini | | | | | | | |

La surface spécifique BET (SBET en m2/g) est déterminée par adsorption d'azote conformément à la norme ASTM-D-3663-78 établie à partir de la méthode BRUNAUER-EMMETT-TELLER décrite dans le périodique "The Journal of American Society", 1938, 60, 309.

Le volume microporeux est déterminé par la méthode t-plot, en utilisant la méthode de Harkins-Jura, avec une régression linéaire du t-plot dans la gamme entre t = 0,35 et 0,5 nm. Le volume poreux total correspond au volume adsorbé à saturation des pores (pression relative d'N2 de 0.99). Le volume mésoporeux est la différence entre le volume poreux total et le volume microporeux.

La cristallinité relative de la zéolithe est mesurée par diffraction aux rayons X. Pour évaluer la cristallinité, les raies de diffraction sont intégrées entre les angles 2 theta de 10 et 70°. On calcule le rapport entre l'intégrale avec correction de la ligne de base et sans correction de la ligne de base. Ce rapport est ensuite normalisé par le rapport d'une zéolite référence (la zéolite mère avant traitement).

On entend par acide de Brønsted un composé pouvant libérer un proton H+ dans le milieu réactionnel. On entend par acide de Lewis un composé pouvant accepter un doublet d'électrons.

La concentration des sites acides de Brønsted et des sites acides de Lewis dans la zéolite est mesurée par adsorption de pyridine. La zéolite est d'abord déshydratée sous vide à 450°C et ensuite exposé à la vapeur de pyridine à 150°C. L'excès de pyridine est éliminé sous vide. La concentration de sites acides Bronsted est déterminée par spectroscopie IR en transmission, par l'intégration de la bande à 1540 cm-1 ; la conversion de l'intensité en mol/g est réalisée en appliquant un coefficient d'extinction de 1.65 cm/µmol. La concentration de sites acides Lewis est déterminée par intégration de la bande à 1450 cm-1, en appliquant un coefficient d'extinction de 1.57 µmol/g.

Le Tableau 3 montre que les traitements avec un hydroxyde de tétraalkylammonium conservent bien le volume microporeux de la zéolithe, notamment avec le tétrapropylammonium et le tétrabutylammonium (A3, A4, B3 et B4). Le volume mésoporeux est fortement augmenté dans tous les cas.

La cristallinité est également très bien conservée par le traitement, à l'exception de B1. L'acidité est proche ou supérieure à la zéolithe mère, à l'exception de B1.

Par rapport à l'art antérieur (BC), le traitement sans NaOH et avec des hydroxydes de tetralkylammonium génère des zéolithes avec une acidité accrue.

### Exemple 2 : Mise en oeuvre des solides de l'exemple 1 pour la conversion du fructose en 5-HMF

Dans un ballon tricol de capacité de 100 mL équipé d'un réfrigérant sont chargés 2 g de fructose cristallin et 20 g de DMSO. Le mélange est porté à la température de 90 °C sous agitation. Une fois la température atteinte, 1.3 g de catalyseur (c'est-à-dire de solide préparé selon l'exemple 1) sont ajoutés, ce qui marque le début de la réaction de déshydratation. Le mélange est alors maintenu en température et sous agitation. Des échantillons sont prélevés à différents temps de réaction et analyser par HPLC pour quantifier les concentrations en fructose et en 5-HMF, et déterminer le rendement en HMF et la conversion du fructose.

Les résultats (taux de conversion du fructose et rendement en HMF à respectivement 2h et 6h de réaction) sont rassemblés dans le tableau suivant :

**[Table 3]**

| Solide | Conv_{FRU} à 2h | Rdt_{HMF} à 2h | Conv_{FRU} à 6h | Rdt_{HMF} à 6h |
|---|---|---|---|---|
| A CBV720 | 40% | 8% | 51% | 21% |
| A1 | 42% | 4% | 51% | 17% |
| A2 | 43% | 8% | 54% | 25% |
| A3 | 54% | 23% | 74% | 46% |
| A4 | 53% | 23% | 74% | 47% |
| B CBV760 | 49% | 19% | 65% | 43% |
| B1 | 47% | 8% | 58% | 26% |
| B2 | 47% | 13% | 66% | 33% |
| B3 | 68% | 37% | 88% | 59% |
| B4 | 58% | 29% | 80% | 50% |

Ces résultats démontrent que les zéolithes préparées avec des hydroxydes de tétraalkylammonium convertissent le fructose. La cinétique de la réaction est particulièrement accélérée (taux de conversion du fructose et rendements en HMF supérieurs après 2 et 6h de réaction par rapport aux zéolithes mères) pour les solides préparés avec l'hydroxyde de tétrapropylammonium et l'hydroxyde de tétrabutylammonium (A3, A4, B3 et B4).

## Revendications

1. Procédé de déshydratation d'une charge comprenant un hexose, ledit procédé comprenant les étapes de :
- Préparation d'un catalyseur de déshydratation, par mise en contact d'une zéolithe comprenant une série de canaux dont l'ouverture est supérieure ou égale à 10 atomes d'oxygène (10MR) et présentant un rapport molaire Si/Al d'au moins 10, avec une solution comprenant un composé organique de formule : 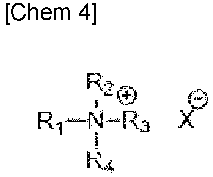 R₁, R₂, R₃ et R₄ étant choisis indépendamment parmi les groupements alkyles linéaires, branchés ou cycliques de 1 à 10 atomes de carbone, et X- étant l'ion hydroxyde OH- ;
- Mise en contact dans au moins un solvant dudit catalyseur de déshydratation avec ladite charge comprenant un hexose, ladite mise en contact étant opérée à une température comprise entre 30 et 160°C et à une pression comprise entre 0,0001 et 8,0 MPa.

2. Procédé selon la revendication 1 dans lequel l'hexose est le fructose ou une unité fructosidique.

3. Procédé selon l'une quelconque des revendications précédentes dans lequel l'ouverture de la série de canaux de la zéolithe est égale à 12 atomes d'oxygène (12MR).

4. Procédé selon la revendication 3 dans lequel la zéolithe est de type structural FAU ou EMT.

5. Procédé selon la revendication 4 dans lequel la zéolithe est une zéolithe USY.

6. Procédé selon l'une quelconque des revendications précédentes dans lequel la zéolithe possède un ratio Si/Al compris entre 10 et 50, de préférence entre 12 et 35.

7. Procédé selon l'une quelconque des revendications précédentes dans lequel le composé organique comprend au moins un groupement choisi parmi méthyl, éthyl, propyl, ou butyl.

8. Procédé selon la revendication 7 dans lequel le composé organique est choisi parmi l'hydroxyde de tétrapropylammonium et l'hydroxyde de tétrabutylammonium.

9. Procédé selon l'une quelconque des revendications précédentes dans lequel la solution comprenant un composé organique ne comprend pas de NaOH.

10. Procédé selon l'une quelconque des revendications précédentes dans lequel le solvant est un solvant aprotique polaire.
